(19) Europäisches Patentamt
European Patent Office
Office européen des brevets

(11) Publication number: **0 502 501 B1**

# EUROPEAN PATENT SPECIFICATION

(45) Date of publication of patent specification: **11.10.95**   (51) Int. Cl.⁶: **A61N  1/30**, A61H 39/00

(21) Application number: **92103687.7**

(22) Date of filing: **04.03.92**

(54) **Device for applying electric fields and medicament ions to acupuncture points.**

(30) Priority: **05.03.91 CN 91101275**

(43) Date of publication of application:
**09.09.92 Bulletin  92/37**

(45) Publication of the grant of the patent:
**11.10.95 Bulletin  95/41**

(84) Designated Contracting States:
**DE FR GB**

(56) References cited:
**EP-A- 0 318 776
WO-A-86/07269
US-A- 4 981 146**

(73) Proprietor: **JIN TONG (GUANG ZHOU) HEALTH
CARE PRODUCTS Co., Ltd.
3/F, Fu Tong Bldg.,
Jinhua Road 1,
Guang Zhou,
Economic Development Zone
Guang Zhou 510730 (CN)**

(72) Inventor: **Fang, Yongxing
Jia 113, Chaoneixiaojie
Beijing (CN)**

(74) Representative: **Heusler, Wolfgang, Dipl.-Ing.
Dr. Dieter von Bezold
Dipl.-Ing. Peter Schütz
Dipl.-Ing. Wolfgang Heusler
Brienner Strasse 52
D-80333 München (DE)**

**Description**

INTRODUCTION

This invention relates generally to a multifunction treating apparatus, and more particularly to a portable multifunction treating apparatus which can treat many diseases, such as hypertension and hypotension, by an electric means, such as a button cell or other electric bodies, to generate electrical field to influence the electrical potentials of the acupuncture points of a human body with electrical induction and at the same time by medicament stored to diffuse and penetrate to the acupuncture points of skin. The apparatus mainly includes an electric means generating electric field, e.g. a button cell, a spacer made of porosint, medicament used to treat a disease, and a case. The electric means is placed on the spacer in the case, and the medicament is also stored in the case. A cure can be taken simply by attaching the apparatus on the human body of a patient with its side near the spacer against the acupuncture point of the skin.

The apparatus, which can be attached on any acupuncture points or any characteristic points of electric potentials, of a human body without any uncomfortable feeling, has evident curative effect and no side effect.

BACKGROUND OF THE INVENTION

With the raise of living standard, patients suffering from hypertension and hypotension become more and more, and specifically among the middle-aged and aged there is a much higher proportion of persons affected by these diseases. The known means of treating such cardiovascular diseases as hypertension is taking antihypertension medicine orally, which has the advantage of evident blood pressure reduction effect and the disadvantage of certain side effect on the health of the patient from the use of long period. Thus. apparatuses have been disclosed to overcome the disadvantage of said oral antihypertension medicine, which treat diseases by electrically stimulating the acupuncture points of a human body and by medicament ions diffusing and penetrating the skin where the acupuncture points are.

For example, a electric adhesive plaster is disclosed in Chinese patent laid-open No. CN104409A, which is to replace some known external medicaments (including ointment, plaster and adhesive tape) to be mainly used in such surgical treatments as subsidence of a swelling and promotion to the growth of the cell issues of muscles, and which can also treat such internal disease as rheumatism to a certain degree. However, it is not suitable for the treatment of the diseases of internal organs and circulation system.

In Chinese Appl. No. 87214076.8, a hypertension treated apparatus is disclosed, which reaches the aim to reduce the patients' blood pressure by micro electric pulses stimulating the acupuncture points on ears. However, during treatment two electrodes are symmetrically connected to two of said acupuncture points, e.g. two ears, of the body of a patient, who presses a switch every five minutes. A pulse is sent to the acupuncture points automatically when the resistance there is low. Completing a treatment costs half an hour. Therefore the apparatus is inconvenient for patients to use. Moreover it needs longer time to become effective.

It can be seen that known apparatuses applying combination of electric stimulation and diffusion and penetration of medicament ions or applying only electric stimulation, come to the aim of treatment by connecting two electrodes with two symmetric acupuncture points and applying electric power to bring pulses at acupuncture points to influence the electric potentials there.

However, all inventors of known treating apparatuses, which apply electric stimulation, neglect the fact that when a human body suffers from a disease, the micro electric potential of corresponding points and other characteristic points of electric potentials change abnormally, and it will take a period of considerable length to bring the potentials of the corresponding points to return to normal by exerting electric stimuli on the corresponding acupuncture point because the stimulation will be effective only after this point is electrically inducted periodically for a long time; that is to say, only by long-term periodic electric induction on the corresponding acupuncture point do functions of parts of human body return to normal, and initial periodic electric induction can only lessen and the following continual long-term and periodic induction can cure the disease. Furthermore, the electric stimulation shall be to influence the acupuncture point corresponding to the disease and not to bring current on it. Thus, because of said facts, the curative effect of known treating apparatuses with electric stimulation is not so desirable as expected and has no much improvement, and patients have to suffer from trivial and boring process of the treatment.

A treatment device serving as transdermal drug applicator and having a housing, a porous body and an electrical source means is known from WO-A-8607269, Fig. 10.

EP 0 502 501 B1

## SUMMARY OF THE INVENTION

In view of said disadvantages of known treating apparatuses with electric stimulation, the present invention treats patients for their diseases by means of electric induction, which is a brand-new concept and a completely different idea from known apparatuses, to influence the electric potential of the acupuncture point corresponding to the disease.

Accordingly, it is an object of the invention to provide a multifunction treating apparatus to treat diseases by applying electric induction to influence the electric potentials of acupuncture points of a human body, and particularly to provide a treating apparatus without any inconvenience and uncomfortable feeling to treat blood pressure unbalancing, namely, hypertension and hypotension, which also have certain curative effect on other diseases, e.g. to alleviate pectoris angina.

To this end, the invention provides a multifunction treating apparatus which influences the electrical potentials of acupuncture points by means of electric induction. Namely, a assembly, which includes a means, e.g. a button cell, which can generate electrical field and is placed on a spacer made of porosint, is placed at a acupuncture point to influence its micro electric potential.

The apparatus according to the invention includes a case having a cavity at its center, a spacer made of porosint and a means generating electric field. In the cavity of the case, through holes are formed in the bottom; the spacer is placed on the bottom; the means is seated on the spacer and medicaments are stored.

Preferably, a big circular spacer and a small circular spacer are provided in said cavity and solid medicament mixed with penetrating accelerator is stored in the small groove which is formed between said big circular spacer and small circular spacer.

Preferably, blocks of soft porous materials, e.g. sponge materials and foamed plastics, are placed in said cavity and soft oily medicament mixed with penetrating accelerator is injected into said blocks.

Preferably, said spacer made of porosint is a microchannel plate which is mixed with additional trace elements.

Additionally or alternatively, the spacer made of porosint is a glass plant with micro parallel passages and said glass plate is mixed with trace elements.

Additionally or alternatively, the spacer made of porosint is a plastic plate with micro parallel passages and said glass plate is mixed with trace elements.

Additionally or alternatively, the spacer made of porosint is a ceramic plate with micro parallel passage and said ceramic plate is mixed with trace elements.

Preferably, said electrical means generating electrical field is a button cell and the negative pole of said button cell is in contact with said spacer made of porosint.

Preferably, a top plate cover is covered on said cell and said medicament; a step is formed at the upper portion of the case and a watchglasslike cover is attached on the case by cooperating with said step; and among said watchglasslike cover, the circular spacers and the case there is at least one of them to be made of insulant to ensure said button cell to be off-position.

## DETAILED DESCRIPTION OF THE INVENTION

The basic concept of the invention is based on the theory of meridian of Chinese traditional medical science which states that body issues at acupuncture points have low electric resistances, well conductibility and higher electric potentials. According to the invention the electric potentials of acupuncture points can be influenced by applying stimuli of additional electric field and by the acupuncture points of a human body absorbing trace elements, to exert an influence on the microcirculation and nervous system of a human body, which can treat blood pressure unbalancing to free patients' suffering. Moreover, the curative effect can be greatly improved by combination said two treatments with human body absorbing medicament.

Generally, the apparatus according to the invention includes a case having a cavity, a spacer made of porosint and a means generating electric field. In the cavity of the case, through holes are formed in the bottom; the spacer is placed on the bottom; the means is seated on the spacer and medicaments are stored. In practice, when the apparatus is attached on the body of a patient, under the effect of the electrical field of the means, the micro electrical potential point of acupuncture points are influenced by electrical induction to reach the aim of stimulating acupuncture points of the human body, while under the same effect trace elements are released from the wall of micro passages of the spacer and diffuse through the through holes to be absorbed by the human body. Like the trace elements medicament ions also diffuse and are absorbed by the human body.

3

Specifically, the multifunction treating apparatus according to the embodiment of the invention includes a watchlike case, at the central part of which a cavity is formed with a plurality of through holes formed in its bottom, a big circular spacer and a small spacer, which are provided in the cavity , a spacer, which is made of porosint, and a button cell, the electric field of which directly acts on a human body to influence the micro electric potentials of acupuncture points by the means of electrostatic induction. The cavity of the case is divided into a big circular groove, a small groove and a central cavity by the big circular spacer and the small circular spacer . The spacer is placed on the bottom of the central cavity and the button cell is seated on the spacer with its negative pole in contact with the spacer. There is solid medicament, which is mixed with penetrating accelerator, stored in the small circular groove; and some blocks of soft porous materials, such as sponge materials or foamed plastic, into which the oily medicament mixed with the penetrating accelerator is injected, may be provided in the small circular groove in order that the medicament diffuses through small through holes to the outer surface of the case easily and is supplied conveniently. A top plate cover is covered on the button cell and the medicament, and by wax the button cell, the circular spacers, and the top plate cover are sealed and fixed. A step is formed at the upper portion of the cavity of the case and a watchglasslike case cover is attached to the case by cooperating with this step and the wax.

A plurality of parallel micro passages are provided through the spacer made of porosint which can be a kind of microchannel plate, e.g. glass plate, plastic plate and ceramic plate with longitudinal parallel passages. There are a plurality of micro passages in the spacer made of porosint. Under the effect of the electric field of the button cell, trace elements in the wall of the passage will flake off and are absorbed by the human body of a patient, and at the same time a plurality of micro parallel electric field, which is stronger than that in the walls of the passages because of the great difference of the dielectric constants between air and the porosint, is formed in the passage to influence the electric potentials of acupuncture points.

It should be noted that among watchglasslike case cover, circular spacers, and case there is at least one of them to be made of insulating materials so that no alive circuit is formed in the apparatus.

Some of the through holes formed in the bottom of the cavity of the case correspond to the spacer to be the passage for trace elements to diffuse, and the other to the medicament for medicament ions to diffuse. In order to have the trace elements and medicament ions persisted on the skin surface for a longer time to ensure a perfect absorption of the human body a seal step is provide on the rear surface of the case to form a very thin space over skin, in which trace elements and medicament ions are preserved and absorbed by human body.

Additional trace elements may be added to the spacer made of porosint in the process of manufacturing to form ones with more trace elements.

Compared with known means of reducing blood pressure, the apparatus of the invention have improved curative effect without side effects. Under the effect of electric field, trace elements and medicament ions, the electric potentials of acupuncture points can be influenced, microcirculation improved, the cell of the part of the body activated and hypertension treated. Moreover, it is convenient to be used because a patient can attach it on his wrist as a watch.

Further objects and advantages of the invention will appear from the following description taken together with the accompanying drawings.

BRIEF DESCRIPTION OF THE DRAWINGS

Figure 1 is a schematic sectional view showing the structure of the apparatus according to the first embodiment of the invention.

Figure 2 is a schematic bottom view of the apparatus of Fig 1 according to the invention.

Figure 3 is a schematic enlarged longitudinally sectional view showing the spacer made of porosint.

Figure 4 is a schematic sectional view of relative positions of the main parts of the apparatus according to the invention.

Figure 5 is a schematic sectional view of the apparatus according to the second embodiment of the invention.

Figure 6 is a schematic sectional view of the apparatus according to the third embodiment of the invention.

DETAILED DESCRIPTION OF THE PREFERRED EMBODIMENTS

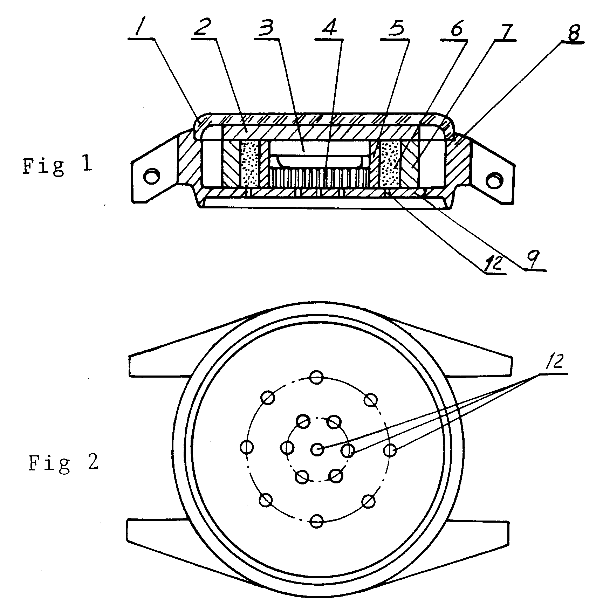

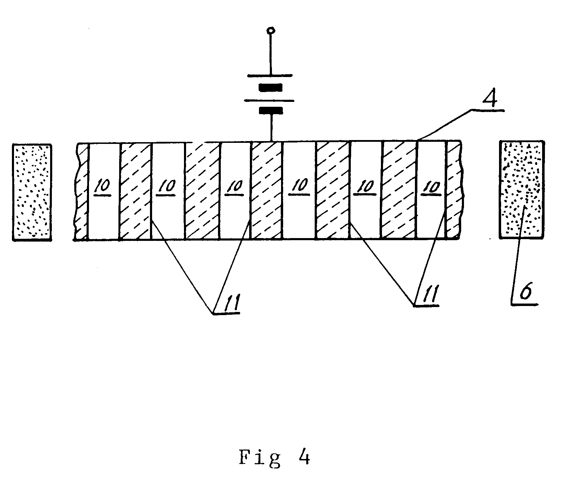

Reference is first made to Fig 1, 2 and 4. The multifunction treating apparatus according to the first embodiment of the invention includes a watchlike case 8, at the central part of which a cavity is formed with a plurality of through holes formed in its bottom 9, a big circular spacer 7 and a small spacer 5, which are provided in the cavity , a spacer 4, which is made of porosint , and a button cell 3, the electric field of which directly acts on a human body to influence the micro electric potentials of acupuncture points by means of electrostatic induction. The cavity of the case 8 is divided into a big circular groove, a small groove and a central cavity by the big circular spacer 7 and the small circular spacer 5. The spacer 4 is placed on the bottom of the central cavity and the button cell 3 is seated on the spacer 4 with its negative pole in contact with the spacer. There is solid medicament 6, which is mixed with penetrating accelerator, stored in the small circular groove; and some blocks of soft porous materials, such as sponge materials or foamed plastic, into which the oily medicament mixed with the penetrating accelerator is injected, may be provided in the small circular groove in order that the medicament 6 diffuses through small through holes to the outer surface of the case 8 easily and is supplied conveniently. A top plate cover 2 is covered on the button cell 3 and the medicament 6, and by wax the button cell 3, the circular spacer 5 and 7, and the top plate cover 7 are sealed and fixed. A step is formed at the upper portion of the cavity of the case 8 and a watchglasslike case cover 1 is attached to the case by cooperating with this step and the wax.

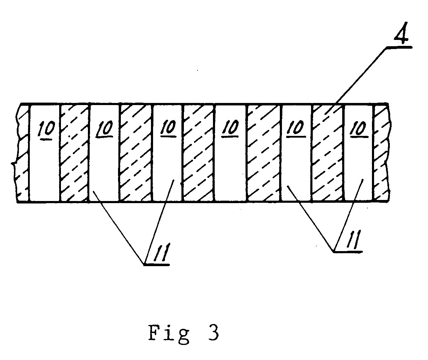

The spacer 4 is made of porosint, which term denotes a body containing a plurality of parallel channels between opposite surfaces. It may be produced by agglutinating a plurality of glass, plastic or ceramic micro tubes which are arranged in parallel, and then cutting the agglomerate into pieces at right angle to the longitudinal axes of the tubes. As shown in Fig. 3, a plurality of parallel micro passages 10 are provided through the spacer 4 which can be a kind of microchannel plate e.g. made of glass, plastic or ceramic with longitudinal parallel passages. Under the effect of the electric field of the button cell 3, trace elements in the wall 11 of the passage 10 will flake off and are absorbed by the human body of a patient, and at the same time a plurality of parallel electric micro fields, which are stronger than that in the walls 11 of the passages 10 because of the great difference of the dielectric constants between air and porosint, is formed in the passage to influence the electric potentials of acupuncture points.

It should be noted that among watchglasslike case cover 1, circular spacers 5 and 7, and case 8 there is at least one of them to be made of insulating materials so that no alive circuit is formed in the apparatus.

Some of the through holes 12 formed in the bottom 9 of the cavity of the case 8 correspond to the spacer 4 to be the passage for trace elements to diffuse, and the other to the medicament for medicament ions to diffuse. In order to have the trace elements and medicament ions persisted on the skin surface for a longer time to ensure a perfect absorption of the human body a seal step is provide on the rear surface of the case 8 to form a very thin space over skin, in which trace elements and medicament ions are preserved and absorbed by human body.

Additional trace elements may be added to the spacer 4 made of porosint in the process of manufacturing to form ones with more trace elements.

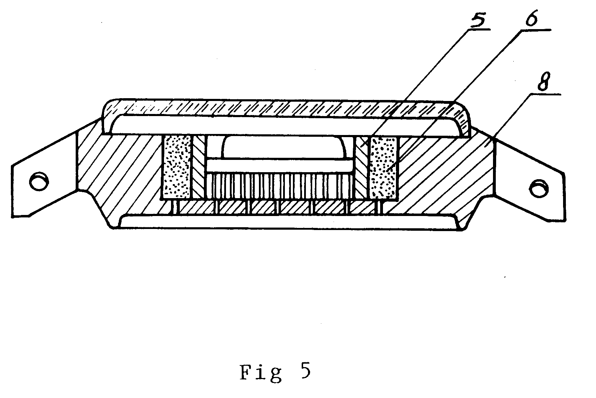

Figure 5 shows the second embodiment according to the invention with the difference from the first one only in that the big circular spacer 7 is not provided in the case.

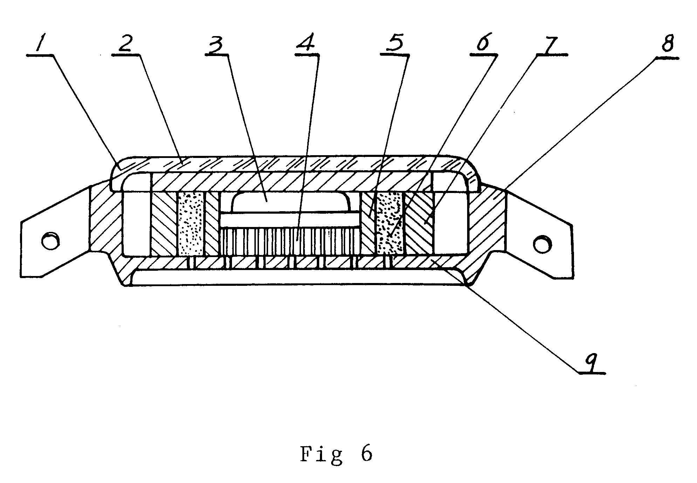

Figure 6 shows the third embodiment of the invention with the difference from the first one only in that the button cell 3 is seated on the spacer 4 with the positive pole in contact with the spacer.

The apparatus with said structure according to the invention can treat diseases with desirable efficacy. The following is a clinical observations of antihypertensive effects of said apparatus.

This observation was a double-blind, randomized, multicenter, parallel group comparison between multifunction treating apparatus (MTA) and captopril treatment.

METHOD

Eligible patients, male or female, at the age ranging from 30 to 60, with stage I or II essential hypertension were randomly selected for treatment for four weeks after completion of one week single-blind placebo run-in period. Those who had a systolic blood pressure equal to or higher than 160mmHG and/or diastolic blood pressure equal to or higher than 95mmHG after one week single-blind placebo period, were eligible for entry into the double-blind period only patients without complicated essential hypertension, free of other severe diseases, were studied.

The following tests were conducted at the entry and end of the study: serum biochemistry (glucose, cholesterol, creatine, SGPT and TTT), haematological indexes (haemoglobin, total WBC count), routine urinalysis, chest radiograph and ECG. After that, the patients were divided into two groups, The first group,

5

called MTA group, was given MTA treatment while taking placebo orally and the second group, called Captopril group, was given captopril treatment while wearing placebos. During treatment the MTA or its corresponding placebos was secured to the patient's wrist on the right Neiguan point of his wrist.

Sitting and standing blood pressure were measured after the patients wore the MTAs or corresponding placebos for the first time for 20 minutes. Then the patients should wear the MTAs or th placeboes twice a day: at 8:30--10:00 am and 3:30--5:00 pm for 20 minutes each time. Captopril or corresponding placebo was taken respectively at the same time . Sitting and standing blood pressure were measured twice a week. Dosages was adjusted for captopril or corresponding placebo from 25mg to 50 mg or more per day. If, at th end of the first week, the goal BP was not achieved, then the dosage could be increased to 75mg per day. In addition to BP, heart rate, drug compliance and adverse effects were also recorded.

The mean of the two measurements at the end of run-in period was taken as the basal BP and the mean of the two measurements after the fourth week was taken as the final BP. The positive response to treatment was proved by the attainment of sitting DBP of less than 95mmHG or a decrease of DBP of equal to or higher than 10mmHG or a decrease of SBP of equal to or higher than 30mmHG. The mean of the final BP and the mean of the first measurement after MTA treatment for 20 minutes were compared with the mean of the basal BP to evaluate MTA curative efficacy. The different efficacies between MTA group and captopril group were also evaluated.

116 patients took part in the double-blind phase, clinical characteristics of the patients for the two groups are shown in Table I.

The final does administered for captopril were 25mg (n = 29), 50mg (n = 22) and 75mg (n = 7). The mean of daily does treatment was 40.5mg.

RESULT AND RESPONSE RATE

The following response rates were obtained after 4 weeks of double-blind therapy for MTA 72.4 percent and for captopril group 74.1 percent. Statistically, there was not any significant difference between the results.

Changes in BP for the two groups are shown in Table II. At first the immediate efficacy of the MTA was evaluated . It was based on measurements made at the end of the placebo run-in period ( baseline) and at 20 minutes after the MTA or captopril administration for the first time on the first day of the double-blind period.

Sitting BP of the patients in the MTA group was reduced from 167.8±18.7/99.4±10.9 mmHG to 159.4±19.7/94.6±10.8 mmHG. The mean reduction was 8.4±10.1/4.8±5.9 mmHG (P<0.001). The BP of the captopril group decreased from 164.1±16.8/99.2±7.6mmHG to 153.5±16.2/95.9±8.1mmHG. The mean reduction was 10.6±11.4/3.3±5.7 mmHG (P<0.01). The differences between two results were of no statistical significance.

Standing BP of the MTA group was reduced from 169.2±21.8/102.3±12.1 mmHG to 160.7±20.2/96.8±11.9 mmHG. The mean reductions were 8.5±10.3/5.5±7.9 mmHG (P<0.001). The standing BP of the captopril group decreased from 166.5±18.7/103.6±8.6 mmHG to 155.5±18.9/97.8±9.7 mmHG. The mean reductions were 10.9±12.5 mmHG (P<0.001) and 5.8±8.4 mmHG (P<0.001). Both differences were of no statistical significance.

A comparison of baseline BP and final BP was conducted. At the same time, a comparison of the results was also conducted.

The sitting and standing BP were reduced respectively from 167.8±18.7/99.4±10.9 mmHG to 151.5±15.9/91.4±9.5 mmHG (P<0.001) and from 169.2±21.8/102.3±12.1 mmHG to 152.0±16.0/92.9±9.2 mmHG (P<0.001) for the MTA group; and the sitting and standing BP of the captopril group decreased respectively from 164.1±16.8/99.2±7.6 mmHG to 149.2±17.3/91.1±8.7 mmHG (P<0.001) and form 166.5±18.7/103.6±8.6mmHG to 149.4±15.2/93. 1±7. 9mmHG (P<0.001). The difference between the baseline BP and final BP in two groups were statistically significant. The difference between two treatment groups were not statistically significant.

There was no change in heart rate and the results of above mentioned lab tests before and after the treatment.

COMPLIANCE

Patients' compliance was good in the study. Only one patient in MTA group withdrew due to noncompliance.

SAFETY

The side effects were considered by the investigators to be possibly or definitely related to the double-blind treatment. The number of patients with side effects was 1 of 58 for MTA group and 4 of 58 for captopril group. Four patients receiving the MTA and captopril treatment experienced slight-systemic itching ( one MTA patient and 3 captopril patients). There was 1 case with nausea and vomiting in the captopril group.

CONCLUSION

MTA can significantly reduce SBP and DBP in both sitting and standing positions. The response rate with MTA was 72.4% after 4 weeks of therapy . The efficacy of the MTA was almost the same as that of captopril taken 25-75mg per day (mean dosage being 40.5mg).
There is few side effects, MTA is indicated the treatment of mild and moderate hypertension.

Table I

| Basic Characteristics of Patients Studied | | |
|---|---|---|
| | MTA (n = 58) | Captopril (n = 58) |
| No M/F | 29/29 | 28/30 |
| Age(yrs.) | 51.5±6.6 | 50.1±7.4 |
| WHO stage I/II | 27/31 | 29/29 |
| Duration of | 9.5±8.2 | 8.1±6.0 |
| Hypertension (yrs.) | (0.5--30) | (0.5--20) |

Table II    Summary of BP Changes (mmHG)  from  Baseline  to Final

| | MTA (n=58) | Captopril (n=58) |
|---|---|---|
| SBP(mmHG) Sitting | | |
| Baseline | 167.8±18.7 | 164.1±16.8 |
| First Administration | 159.4±19.7*** | 153.5±16.2** |
| Final | 151.5±15.9* | 149.2±17.3* |
| Standing | | |
| Baseline | 169.2±21.8 | 166.5±18.7 |
| First Administration | 160.7±20.2*** | 155.5±18.9** |
| Final | 152.0±16.0* | 149.4±15.2* |
| DBP(mmHG) Sitting | | |
| Baseline | 99.4±10.9 | 99.2±7.6 |
| First Administration | 94.6±10.8*** | 95.9±8.1** |
| Final | 91.4±9.5* | 91.1±8.7* |
| Standing | | |
| Baseline | 102.3±12.1 | 103.6±8.6 |
| First Administration | 96.8±11.9 | 97.8±9.7** |
| Final | 92.9±9.2* | 93.1±7.9* |

*: P<0.001. **: P<0.01, ***: P<0.001(comparison to baseline)

EP 0 502 501 B1

## Claims

1. A multifunction treating apparatus particularly for treating diseases, said apparatus including:
   a case (8) at the central part of which a cavity is formed with a plurality of through holes (12) provided in its bottom (9);
   a spacer (4) which is made of a porous body and is placed on the bottom of the cavity;
   an electrical means (3) which generates an electrical field and is seated on said spacer (4); and
   a medicament (6) which is stored in the cavity;
   the apparatus being insulated so that there is no current flowing in a circuit closed via the patient's skin.

2. A multifunction treating apparatus as in claim 1, wherein a big circular spacer (7) and a small circular spacer (5) are provided in said cavity and solid medicament (6) mixed with penetrating accelerator is stored in the small groove which is formed between said big circular spacer (7) and small circular spacer (5).

3. A multifunction treating apparatus as in claim 2, wherein blocks of soft porous materials, e.g. sponge materials and foamed plastics, are placed in said cavity and soft oily medicament (6) mixed with penetrating accelerator is injected into said blocks.

4. A multifunction treating apparatus as in claim 1 wherein a small circular spacer (5) is provided in said cavity and medicament mixed with penetrating accelerator is stored in the circular groove which is formed between the wall of said cavity and the small circular spacer (5).

5. A multifunction treating apparatus as in claim 2, 3 or 4, wherein said spacer (4) made of porosint is a microchannel plate which is mixed with additional trace elements.

6. A multifunction treating apparatus as in claim 5 wherein said spacer (4) made of porosint is a glass plate with micro parallel passages (10) and said glass plate is mixed with trace elements.

7. A multifunction treating apparatus as in claim 5 wherein said spacer (4) made of porosint is a plastic plate with micro parallel passages (10) and said plate is mixed with trace elements.

8. A multifunction treating apparatus as in claim 5 wherein said spacer (4) made of porosint is a ceramic plate with micro parallel passages (10) and said ceramic plate is mixed with trace elements.

9. A multifunction treating apparatus as in claims 6, 7 or 8 wherein said electrical means generating electrical field is a button cell (3) and the negative pole of said button cell (3) is in contact with said spacer (4) made of porosint.

10. A multifunction treating apparatus as claims in 6, 7, or 8 wherein the positive pole of a button cell (3) is in contact with said spacer (4) made of porosint.

11. A multifunction treating apparatus as in claim 9 wherein a top plate cover (2) is covered on said cell (3) and said medicament (6); a step is formed at the upper portion of the case and a watchglass like cover (1) is attached on the case (8) by cooperating with said step; and among said watchglass like cover (1), said circular spacer (5,7) and said case (8) there is at least one of them to be made of insulant to ensure said button cell (3) to be off-position.

12. A multifunction treating apparatus as in claim 1 wherein said case (8) is shaped like the case of a wrist watch.

## Patentansprüche

1. Mehrfunktions-Behandlungsvorrichtung, insbesondere zum Behandeln von Krankheiten, enthaltend:
   ein Gehäuse (8), in dessen mittlerem Teil ein Hohlraum mit einer Vielzahl von in seinem Boden (9) vorgesehenen Durchgangslöchern (12) gebildet ist;
   einen Abstandshalter (4), der aus einem porösen Körper hergestellt und auf dem Boden des

8

Hohlraums angeordnet ist;

eine elekrische Einrichtung (3), die ein elektrisches Feld erzeugt und auf dem Abstandshalter (4) sitzt, und

ein Medikament (6), das als Depot im Hohlraum untergebracht ist,

wobei die Vorrichtung so isoliert ist, daß kein Strom in einem über die Haut des Patienten geschlossenen Stromkreis fließt.

2. Mehrfunktions-Behandlungsvorrichtung nach Anspruch 1, worin ein großer kreisförmiger Abstandshalter (7) und ein kleiner kreisförmiger Abstandshalter (5) im Hohlraum vorgesehen sind und in der kleinen Furche, die zwischen dem großen kreisförmigen Abstandshalter (7) und dem kleinen kreisförmigen Abstandshalter (5) gebildet ist, ein mit einem Eindringbeschleuniger gemischtes Feststoffmedikament (6) deponiert ist.

3. Mehrfunktions-Behandlungsvorrichtung nach Anspruch 2, worin Blöcke aus weichen porösen Stoffen, z.B. Schwammstoffen und geschäumten Kunstsstoffen, im Hohlraum angeordnet sind und ein mit einem Eindringbeschleuniger gemischtes geschmeidiges öliges Medikament (6) in die Blöcke injiziert ist.

4. Mehrfunktions-Behandlungsvorrichtung nach Anspruch 1, worin im Hohlraum ein kleiner kreisförmiger Abstandshalter (5) vorgesehen ist und ein mit einem Eindringbeschleuniger gemischtes Medikament in der kreisförmigen Furche deponiert ist, die zwischen der Wand des Hohlraumes und dem kleinen kreisförmigen Abstandshalter (5) gebildet ist.

5. Mehrfunktions-Behandlungsvorrichtung nach Anspruch 2, 3 oder 4, worin der aus Porosint bestehende Abstandshalter (4) eine Mikrokanal-platte ist, die mit zusätzlichen Spurenelementen gemischt ist.

6. Mehrfunktions-Behandlungsvorrichtung nach Anspruch 5, worin der aus Porosint bestehende Abstandshalter (4) eine Glasplatte mit parallelen Mikrokanälen (10) ist und die Glasplatte mit Spurenelementen gemischt ist.

7. Mehrfunktions-Behandlungsvorrichtung nach Anspruch 5, worin der aus Porosint bestehende Abstandshalter (4) eine Kunststoffplatte mit parallelen Mikrokanälen (10) ist und diese Platte mit Spurenelementen gemischt ist.

8. Mehrfunktions-Behandlungsvorrichtung nach Anspruch 5, worin der aus Porosint bestehende Abstandshalter (4) eine Keramikplatte mit parallelen Mikrokanälen (10) ist und diese Keramikplatte mit Spurenelementen gemischt ist.

9. Mehrfunktions-Behandlungsvorrichtung nach Anspruch 6, 7 oder 8, worin die ein elektrisches Feld erzeugende elektrische Einrichtung eine Knopfzelle (3) ist und der negative Pol dieser Knopfzelle (3) in Kontakt mit dem aus Porosint bestehenden Abstandshalter (4) ist.

10. Mehrfunktions-Behandlungsvorrichtung nach Anspruch 6, 7 oder 8, worin der positive Pol einer Knopfzelle (3) in Berührung mit dem aus Porosint bestehenden Abstandshalter (4) ist.

11. Mehrfunktions-Behandlungsvorrichtung nach Anspruch 9, worin über die Zelle (3) und das Medikament (6) eine obere Deckplatte (2) gelegt ist, im oberen Teil des Gehäuses eine Schulter geformt ist und unter Zusammenwirkung mit dieser Schulter ein uhrglasähnlicher Deckel (1) am Gehäuse (8) befestigt ist und von den Elementen, welche den uhrglasähnlichen Deckel (1), die kreisförmigen Abstandshalter (5, 7) und das Gehäuse (8) umfassen, mindestens eines aus Isolierstoff besteht, um sicherzustellen, daß die Knopfzelle (3) ohne geschlossenen Stromkreis ist.

12. Mehrfunktions-Behandlungsvorrichtung nach Anspruch 1, worin das Gehäuse (8) ähnlich dem Gehäuse einer Armbanduhr gestaltet ist.

**Revendications**

1. Appareil de traitement à fonctions multiples, destiné plus particulièrement au traitement de maladies, comportant a boîtier (8) dans la partie centrale duquel est formée une cavité dont le fond (9) est percé d'une pluralité de trous traversants (12), un organe d'espacement (4) qui est constitué d'un corps poreux et qui est placé sur le fond de la cavité, un moyen électrique (3) qui produit un champ électrique et qui prend appui sur l'organe d'espacement (4), et un médicament (6) qui est stocké dans la cavité, l'appareil état isolé de telle façon qu'aucun courant ne passe dans un circuit fermé par l'intermédiaire de la peau d'un patient.

2. Appareil de traitement à fonctions multiples suivant la revendication 1 caractérisé en ce qu'un grand organe d'espacement circulaire (7) et a petit organe d'espacement circulaire (5) sont prévus dans la cavité et un médicament solide (6), mélangé avec un accélérateur de pénétration, est stocké dans le petit espace qui est formé entre le grand organe d'espacement circulaire (7) et le petit organe d'espacement circulaire (5).

3. Appareil de traitement à fonctions multiples suivant la revendication 3 caractérisé en ce que des blocs de matériaux poreux souples, tels que par exemple des matériaux spongieux et des mousses de matière plastique, sont placés dans la cavité et un médicament huileux mou (6), mélangé avec un accélérateur de pénétration, est injecté dans ces blocs.

4. Appareil de traitement à fonctions multiples suivant la revendication 1 caractérisé en ce qu'un petit organe d'espacement circulaire (5) est prévu dans la cavité et un médicament mélangé avec a accélérateur de pénétration est stocké dans l'espace circulaire qui est formé entre la paroi de la cavité et le petit organe d'espacement circulaire (5).

5. Appareil de traitement à fonctions multiples suivant l'une quelconque des revendications 2,3 ou 4 caractérisé en ce que l'organe d'espacement (4), constitué d'un corps à pores parallèles, est me plaque à microcanaux qui est combinée avec des éléments traces additionnels.

6. Appareil de traitement à fonctions multiples suivant la revendication 5 caractérisé en ce que l'organe d'espacement (4) constitué par un corps a pores parallèles est une plaque de verte avec des micropassages parallèles (10) et cette plaque de verte est combinée avec des éléments traces.

7. Appareil de traitement à fonctions multiples suivant la revendication 5 caractérisé en ce que l'organe d'espacement (4) constitué par un corps à pores parallèles est une plaque de matière plastique avec des micropassages parallèles (10) et cette plaque de matière plastique est combinée avec des éléments traces.

8. Appareil de traitement à fonctions multiples suivant la revendication 5 caractérisé en ce que l'organe d'espacement (4) constitué par un corps à pores parallèles est une plaque en céramique avec des micropassages parallèles (10) et cette plaque en céramique est combinée avec des éléments traces.

9. Appareil de traitement à fonctions multiples suivant l'une quelconque des revendications 6,7 ou 8 caractérisé en ce que le moyen électrique produisant un champ électrique est une pile bouton (3) et le pôle négatif de cette pile bouton (3) est en contact avec l'organe d'espacement (4) constitué par un corps à pores parallèles.

10. Appareil de traitement à fonctions multiples suivant l'une quelconque des revendications 6,7 ou 8 caractérisé en ce que le pôle positif d'une pile bouton (3) est en contact avec l'organe d'espacement (4) constitué par un corps à porcs parallèles.

11. Appareil de traitement à fonctions multiples suivant la revendication 9 caractérisé en ce qu'une plaque couvercle supérieure (2) recouvre la pile (3) et le médicament (6), un épaulement est formé dans la partie supérieure du boîtier et un couvercle (1) du type verte de montre est attaché au boîtier (8) par suite de sa coopération avec l'épaulement, et l'un au moins du couvercle (1) du type verte de montre, des organes d'espacement circulaires (5,7) et du boîtier (8) est réalisé en une matière isolante afin d'assurer que la pile bouton (3) n'est pas mise en circuit fermé.

12. Appareil de traitement à fonctions multiples suivant la revendication 1 caractérisé en ce que le boîtier (8) a la forme du boîtier d'une montre poignet.

Fig 1

Fig 2

Fig 3

Fig 4

EP 0 502 501 B1

Fig 5

Fig 6